(19) Europäisches Patentamt
European Patent Office
Office européen des brevets

(11) Publication number: **0 092 862**
**B1**

(12) # EUROPEAN PATENT SPECIFICATION

(45) Date of publication of patent specification: **12.03.86**

(51) Int. Cl.$^4$: **C 07 C 135/02**

(21) Application number: **83200495.6**

(22) Date of filing: **08.04.83**

(54) **Process for the oxidation of tertiary amines to amine oxides.**

(30) Priority: **21.04.82 US 370646**

(43) Date of publication of application:
**02.11.83 Bulletin 83/44**

(45) Publication of the grant of the patent:
**12.03.86 Bulletin 86/11**

(84) Designated Contracting States:
**BE DE FR GB IT NL**

(58) References cited:

**HOUBEN-WEYL: "Methoden der organischen Chemie", vol. IV/1a Oxidation, Part 1, 1981, Georg Thieme Verlag, Stuttgart, DE.**

(73) Proprietor: **THE PROCTER & GAMBLE COMPANY**
**301 East Sixth Street**
**Cincinnati Ohio 45202 (US)**

(72) Inventor: **Riley, Dennis Patrick**
**1457 Longacre Drive**
**Forest Park Ohio 45240 (US)**
Inventor: **Shumate, Robert Edward**
**9631 Wildbrook Lane**
**Cincinnati Ohio 45231 (US)**

(74) Representative: **Ernst, Hubert et al**
**PROCTER & GAMBLE EUROPEAN TECHNICAL CENTER Temselaan 100**
**B-1820 Strombeek-Bever (BE)**

**EP 0 092 862 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical field

The present application relates to the preparation of amine oxides, especially those amine oxides suitable as detergent surfactants, by the oxidation of nonaromatic tertiary amines with molecular oxygen.

Amine oxides have a number of different uses. For example, amine oxides prepared from aromatic and nonaromatic tertiary amines can be used as light duty liquid detergents, pour point depressants and polymerization inhibitors. See U.S. Patent 3,657,251 to Smetana, issued April 18, 1972. Amine oxides have also been used in other areas such as the drug field. See U.S. Patent 3,520,888 to Johnston, issued July 21, 1970, which discloses 1,2,3,4 - tetrahydrophenazine - 5,10 - dioxides useful in controlling chronic respiratory disease in poultry and in promoting and improving feed efficiency of animals in general.

Of particular importance is the use of amine oxides, especially the nonaromatic variety, as detergent surfactants. Detergent compositions containing these surfactant amine oxides exhibit superior performance in cool or cold water laundering operations. Such amine oxides are usually derived from trialkyl tertiary amines or monoalkylalkylene oxide dialkyl tertiary amines. One of the alkyl groups (or the monoalkylalkylene oxide group) usually has a relatively long carbon chain (e.g., $C_{10}$—$C_{18}$) while the remaining two alkyl groups have relatively short carbon chains (e.g., $C_1$—$C_4$). See, for example, U.S. Patent 4,276,205 to Ferry, issued June 30, 1981 (amine oxides derived from trialkyl or monoalkylalkylene oxide dialkyl tertiary amines suitable for cool or cold water detergents); U.S. Patent 3,843,563 to Davies et al., issued October 22, 1974 (detergent composition containing amine oxides derived from trialkyl tertiary amines); U.S. Patent 3,341,459 to Davis, issued September 12, 1967 (amine oxides derived from alkylpolyethoxy dialkyl tertiary amines suitable for cool water detergent compositions). Hydroxy groups can be substituted at the 2 or 3 position of one or more of the alkyl groups to lower the degree of hygroscopicity and to improve the thermal stability of the detergent composition. See, for example, U S Patent 3,202,714 to Zimmerer et al., issued August 24, 1965; U.S. Patent 3,441,611 to Drew et al., issued April 29, 1969.

Amine oxides can be prepared by oxidation of the respective tertiary amines with a strong oxidizing agent. The preferred oxidizing agent used is hydrogen peroxide. A dilute, or preferably concentrated (30% or greater) hydrogen peroxide solution is added in a stoichiometric or greater amount to an aqueous solution containing the tertiary amine for conversion thereof to the amine oxide. See U.S. Patent 3,215,741 to Chadwick, issued November 2, 1965; U.S. Patent 3,432,555 to Mahnken, issued March 11, 1969; U.S. Patent 3,463,817 to Mahnken, issued August 26, 1969. The yields and reaction rate can be improved by incorporation of catalysts and/or chelating agents. See U.S. Patent 3,333,000 to Albert et al., issued July 25, 1967 (mixture of an alkali metal carbonate and an alkali metal polyphosphate as a catalyst); U.S. Patent 4,247,480 to Murata et al., issued January 27, 1981 (carbon dioxide as a catalyst); U.S. Patent 3,283,007 to Chadwick, issued November 1, 1966 (diethylenetriaminepentaacetic acid as a chelating agent). Other oxidizing agents such as the peroxy acids and ozone have also been used in oxidizing tertiary amines to the respective amine oxides. See Cope et al., "Rearrangement of Allyldialkylamine Oxides and Benzyldimethyl amine Oxide," *J. Am. Chem. Soc.*, Vol. 71, (1949), pp. 3423—28 (oxidation of allyldi-n-hexyl amine with perbenzoic acid to the respective amine oxide); Wragg et al., "The Rearrangement of Amine Oxides *Chem. Soc.*, (1958), pp. 4057—64 (oxidation of benzylmethylaniline and allylmethylaniline to the respective amine oxides using monoperphthalic acid); U.S. Patent 3,520,888 to Johnston, issued July 21, 1970 (oxidation of 1,2,3,4 - tetrahydrophenazines to the respective 5,10-dioxides using peracetic acid, perbenzoic acid, m-chloroperbenzoic acid, performic acid or monoperphthalic acid); U.S. Patent 3,657,251 to Smetana, issued April 18, 1972 (oxidation of tertiary amines to amine oxides using an ozone derivative in the presence of a molybdenum, tungsten or vanadium oxide forming catalyst; oxidation of tertiary amines to amine oxides using peracetic acid, perbenzoic acid and monoperphthalic acid also disclosed); Hentges et al., "Amine Oxidation: The Reaction of Tri-n-butylamine with Ozone", *J. Chem. Soc.*, (1964), pp. 711—714 (oxidation of tri-n-butylamine to the respective amine oxide by ozone in chloroform or methanol).

The primary process for preparing amine oxides used in detergent compositions is by oxidation of the respective tertiary amines with hydrogen peroxide. However, the use of hydrogen peroxide as an oxidizing agent has a number of significant disadvantages. Compared to oxygen, hydrogen peroxide is a relatively expensive oxidizing agent. Also, as with any strong oxidizing agent, hydrogen peroxide requires special handling. Further, the oxidation of longer chain alkyl tertiary amines by hydrogen peroxide can cause gel formation problems unless the hydrogen peroxide concentration is dilute, the reaction temperature is controlled, salts are added to the reaction mixture, or the reaction mixture is diluted with water during the course of the reaction. See U.S. Patent 3,432,555 to Mahnken, issued March 11, 1969 (col. 1, line 39 to col. 2, line 40); U.S. Patent 3,463,817 to Mahnken, issued August 26, 1969 (col. 1, line 43 to col. 2, line 66). U S Patent 3,215,741 to Chadwick, issued November 2, 1965 (col. 1. line 39 to col. 2, line 22).

Besides the use of strong oxidizing agents, little attention has been paid to other methods for conversion of tertiary amines to amine oxides, especially those using oxygen or air as the oxidizing agent Fayter et al., "Chemistry of Ultrasound: The Irradiative Behavior of Simple Aliphatic Amines", *J. Acous Soc. Am.*, Vol. 56, (1974), pp. 1461—68, discloses a method for conversion of trimethylamine to the respective amine oxide using oxygen. This method involves ultrasonic irradiation of a 0.08 *M* aqueous solution of trimethylamine under a positive flow of oxygen at 800 kHz for 4.5 hours at a temperature of

21°C. From Table I at page 1462 of the Fayter et al. article, the yield of the amine oxide from this method was only about 15%. A significant percentage (about 13%) of other reaction products such as ammonia, dimethylamine, methyl alcohol and formaldehyde were formed as well. Ultrasonic irradiation as a method for conversion of tertiary amines to amine oxides is also a fairly exotic process which would probably not be suitable in a commercial operation for conversion of nonaromatic tertiary amines to amine oxides suitable in detergent compositions.

It is therefore an object of the present invention to provide a process for conversion of nonaromatic tertiary amines to the respective amine oxides which does not require expensive oxidizing agents such as hydrogen peroxide or peroxyacids.

It is another object of the present invention to provide a process for conversion of nonaromatic tertiary amines to the respective amine oxides which does not require an oxidation catalyst.

It is a further object of the present invention to provide a process for conversion of nonaromatic tertiary amines to the respective amine oxides using molecular oxygen as the oxidizing agent.

It is yet another object of the present invention to provide a process for conversion of nonaromatic tertiary amines to the respective amine oxides in relatively high yields without significant formation of other reaction products.

It is yet a further object of the present invention to provide a process for conversion of nonaromatic tertiary amines to detergent surfactant amine oxides on a commercial scale.

These and further objects of the present invention are hereinafter disclosed.

Other background art

Barton et al, *Comp. Org. Chem.*, Vol. 1, (1979), p. 201, discloses that amine oxides are easily prepared in high yield from the corresponding tertiary amines by a variety of oxidants including hydrogen peroxide, various peroxyacids and ozone. Exhaustive alkylation of hydroxylamine is also disclosed as a method for preparing low molecular weight, symmetrical amine oxides.

U.S. Patent 4,072,706 to Hershman et al., issued February 7, 1978, discloses oxidation of tertiary phosphonomethylamines with oxygen to cause cleavage of the phosphonomethyl group and selective production of the secondary amine.

Smith et al., "Electrochemical Dealkylation of Aliphatic Amines", *J. Org. Chem.*, Vol. 34, (1969), pp. 1821—26, discloses the anodic oxidation of tertiary amines, in particular, tri-n-propylamine, in nonaqueous systems to produce a secondary amine and an aldehyde. Introduction of oxygen caused formation of N,N-dipropylformamide. See paragraph bridging pp. 1824—1825.

Galliani et al., "Horseradish Peroxidase-catalysed Oxidation of Aromatic Tertiary Amines with Hydrogen Peroxide", *J. Chem. Soc. (Perkin I)*, (1978), pp. 456—60, discloses that horseradish peroxidase catalyzed oxidation of aromatic tertiary amines with hydrogen peroxide leads to a secondary amine and an aldehyde. This article states that there was no evidence for the formation of amine oxides. See p. 457.

Fatiadi, "Active Manganese Dioxide Oxidation in Organic Chemistry", *Synthesis*, (1976), pp. 133—67, discloses that tertiary amines are readily oxidized by a variety of reagents, the initial products being amine oxides, enamines or carbinolamines. See p. 147.

Oswald et al., "Organic Nitrogen Compounds:Peroxide Intermediates of Tertiary Alkylamine Oxidation by Hydrogen Peroxide", *J. Org. Chem.*, Vol. 28, (1963), pp. 651—57, discloses that oxidation of tertiary aliphatic amines by aqueous hydrogen peroxide to yield amine oxides is a well known reaction.

Disclosure of the invention

The present invention relates to a novel process for the oxidation of nonaromatic tertiary amines to the respective amine oxides using molecular oxygen. An aqueous solvent system containing the nonaromatic tertiary amine is provided with an initial pH about equal to or greater than the pKa of the tertiary amine. In the absence of an oxidation catalyst, this aqueous system is contacted with molecular oxygen at an oxygen partial pressure of at least 50 psi (3.45 $10^5$ Pa) and a temperature of at least 80°C. The resulting oxidation converts the tertiary amine to the respective amine oxide.

The process of the present invention results in the conversion of the tertiary amine to the respective amine oxide at fairly high yields, usually without significant formation of other reaction products. Depending upon the duration of the reaction, yields of at least 40%, and more typically at least 80%, can be achieved using this process. Surprisingly, these high yields are achieved with a source of molecular oxygen such as air, rather than a more expensive oxidizing agent such as hydrogen peroxide. Such high yields are also surprisingly achieved without the need of an oxidation catalyst and by using fairly moderate temperatures and pressures. In particular, the process of the present invention can be used to convert nonaromatic tertiary amines to detergent surfactant amine oxides on a commercial scale.

Detailed description of the invention

A. Tertiary amines suitable for conversion to amine oxides

The process of the present application is suitable for the conversion of a variety of nonaromatic tertiary amines to the respective amine oxides. As used herein, the term "nonaromatic tertiary amine" refers to both noncyclic tertiary amines and nonaromatic cyclic tertiary amines. It has been found that the process of the present application does not convert aromatic cyclic tertiary amines to the respective amine oxides.

Examples of such aromatic tertiary amines include the pyridines, pyrazines, pyrroles, purines, pyrimidines and like compounds.

As used herein, the term "noncyclic tertiary amine" refers to tertiary amines having the following general formula:

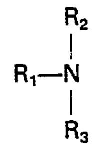

wherein $R_1$, $R_2$ and $R_3$ are nonaromatic groups (e.g., alkyl) or aromatic groups which are not directly substituted on the nitrogen atom (e.g., benzyl). It has been found that noncyclic tertiary amines with aromatic groups (e.g., phenyl) which are directly substituted on the nitrogen atom are not converted by the process of the present application to the respective amine oxides. Examples of such tertiary amines with directly substituted aromatic groups include N,N-dimethylaniline (but not N,N-dimethylbenzylamine), N,N-methylphenylaniline and like compounds.

In this general formula, $R_1$, $R_2$ and $R_3$ can all be the same substituent or can be different substituents. Examples of the former class of noncyclic tertiary amines include trimethylamine, triethylamine, tri-n-propylamine, and tri-n-butylamine. Examples of the latter class include dimethyldodecylamine and dimethyltetradecylamine.

One class of noncyclic tertiary amines for which the process of the present application is particularly suitable are those having the following general formula:

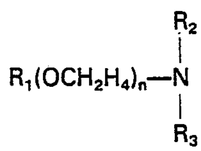

wherein $R_1$ is a $C_{10}$—$C_{22}$ (typically $C_{10}$—$C_{18}$) hydrocarbyl group and n is from 0 to about 10; and $R_2$ and $R_3$ are each a $C_1$—$C_4$ alkyl group. For the $R_1$ substituent, the hydrocarbyl group can be a straight- or branched-chain alkyl, alkaryl (e.g., alkylphenyl or alkylbenzyl), or a substituted hydrocarbyl group (e.g., hydroxyalkyl). For the $R_2$ and $R_3$ substituents, the alkyl group can be a straight- or branched-chain alkyl or substituted alkyl (e.g., hydroxyalkyl). Included within this class are the trialkyl tertiary amines or monoalkylalkylene oxide dialkyl tertiary amines which can be converted by the process of the present application to detergent amine oxides disclosed in, for example, U.S. Patent 4,276,205 to Ferry, issued June 30, 1981 (herein incorporated by reference); U.S. Patent 3,843,563 to Davies et al., issued October 22, 1974 (herein incorporated by reference); and U.S. Patent 3,341,459 to Davis, issued September 12, 1967 (herein incorporated by reference). Also included are those noncyclic tertiary amines having hydroxyalkyl groups which can be converted by the process of the present application to the detergent amine oxides disclosed in, for example, U.S. Patent 3,202,714 to Zimmerer et al., issued August 24, 1965 (herein incorporated by reference) and U.S. Patent 3,441,611 to Drew et al., issued April 29, 1969 (herein incorporated by reference). Specific examples of such tertiary amines include dimethyldodecylamine, dimethyltetradecylamine, ethylmethyltetradecylamine, dimethylcetylamine, dimethylstearylamine, ethylpropylcetylamine, diethyldodecylamine, diethyltetradecylamine, dipropyldodecylamine, bis - (2 - hydroxyethyl) dodecylamine, bis - (2 - hydroxyethyl) - 3 - dodecoxy - 2 - hydroxypropylamine, (2 - hydroxypropyl)methyltetradecylamine, dimethyl - (2 - hydroxydodecyl)amine, and the corresponding decyl, hexadecyl and octadecyl homologs of these amines. Preferred amines herein are the $C_{12}$—$C_{14}$ dimethylamines. Particularly preferred is dimethyldodecylamine.

Another class of noncyclic tertiary amines for which the process of the present application is suitable are those which can be converted to the alpha-amine oxide detergent surfactants (carboxylic acids or salts thereof having amine oxide substituents at the alpha-carbon atom) disclosed in European Patent Application No. 0 060 710, to James F. Ward, published on September 22, 1982 (herein incorporated by reference). These tertiary amines (hereinafter alpha tertiary amines) have the following formula:

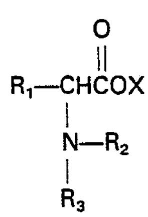

wherein $R_1$ is hydrogen or a $C_1$—$C_{20}$ hydrocarbyl group; $R_2$ and $R_3$ are each a $C_1$—$C_{20}$ alkyl group, or a $C_2$—$C_3$ alkylene oxide group having from 1 to 10 alkylene oxide units; and X is hydrogen or a water-soluble

metal, ammonium or substituted ammonium cation; the total number of carbon atoms for the hydrocarbyl and alkyl groups of $R_1$, $R_2$ and $R_3$ being from 8 to 36.

In the above formula, the $R_1$ hydrocarbyl group can be a straight or branched chain alkyl, alkaryl (e.g., alkylphenyl or alkylbenzyl), or a substituted hydrocarbyl group (e.g., hydroxyalkyl). The nature of $R_1$ can be varied by the selection of the parent carboxylic acid. Typical carboxylic acid starting materials include acetic acid, propionic acid, butyric acid, valeric acid, caproic acid, caprylic acid, capric acid, lauric acid, myristic acid, palmitic acid, stearic acid, eicosanoic acid, mixed coconut oil fatty acids, mixed palm oil fatty acids, mixed lard fatty acids, and mixed tallow fatty acids, which are preferred for cost considerations. $R_1$ is preferably a $C_8$—$C_{20}$ hydrocarbyl group, and most preferably a $C_{10}$—$C_{16}$ alkyl group.

The $R_2$ and $R_3$ substituents can be an alkyl group (straight or branched chain alkyl or substituted alkyl, e.g., hydroxyalkyl), or a $C_2$—$C_3$ alkylene, preferably ethylene, oxide group containing from 1 to 10, preferably 1 to 5, alkylene oxide units. Such a $C_2$—$C_3$ alkylene oxide group would commonly, and preferably, be terminated with a hydrogen atom, but also can be terminated with a methyl, ethyl or propyl group. $R_2$ and $R_3$ are preferably a $C_1$—$C_4$ alkyl group, and more preferably a methyl, ethyl, 2-hydroxyethyl or 2-hydroxypropyl group.

For substituent X, suitable water-soluble metal cations include any of the alkali metal (e.g. sodium, potassium) and alkaline earth metal (e.g. calcium, magnesium) cations. Useful substituted ammonium cations include, for example, the methyl-, dimethyl-, trimethyl-, diethanol- and triethanolammonium cations and quaternary ammonium cations such as tetramethylammonium and dimethyl piperidinium cations. Preferably X is a water-soluble alkali metal cation. Most preferably, X is sodium.

It will be appreciated that the above substituents are selected such that the respective amine oxides exhibit sufficient surface activity and solubility for their intended use. Thus, the total number of carbon atoms for the hydrocarbyl and alkyl groups of the $R_1$, $R_2$ and $R_3$ substituents are from 8 to 36, preferably from 12 to 26. Additionally, when $R_1$, one of $R_2$ and $R_3$, have relatively long hydrocarbyl or alkyl chains, it is preferred that the other $R_2$ or $R_3$ be a $C_2$—$C_3$ alkylene (preferably ethylene) oxide group for optimum solubility, especially in cold water.

Another class of noncyclic tertiary amines for which the process of the present application is suitable are those tertiary amines which can be converted to the alpha-oxyalkylene amine oxide detergent surfactants (carboxylic acids, or salts, esters or amides thereof having an oxyalkylene amine oxide substituent at the alpha-carbon atom) disclosed in European Patent Application No. 0 060 712, to Eugene P. Gosselink, published on September 22, 1982 (herein incorporated by reference). These tertiary amines (hereafter alpha oxyalkylene tertiary amines) have the following formula:

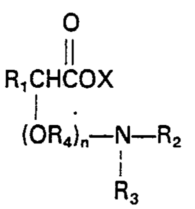

or

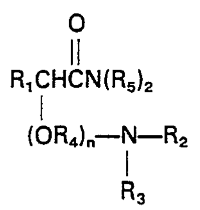

wherein $R_1$ is hydrogen or a $C_1$—$C_{20}$ hydrocarbyl group; $R_2$ and $R_3$ are each a $C_1$—$C_{20}$ alkyl group, or a $C_2$—$C_3$ alkylene oxide group having from 1 to about 10 alkylene oxide units; $R_4$ is a $C_2$—$C_6$ alkylene group, and n is from 1 to 20; each $R_5$ is hydrogen, a $C_1$—$C_{20}$ hydrocarbyl group, or a $C_2$—$C_3$ alkylene oxide group having from 1 to 10 alkylene oxide units; and X is hydrogen, a water-soluble metal, ammonium or substituted ammonium cation, a $C_1$—$C_8$ hydrocarbyl group, or a $C_2$—$C_3$ alkylene oxide group having from 1 to 10 alkylene oxide units; the total number of carbon atoms for the hydrocarbyl and alkyl groups of $R_1$, $R_2$, $R_3$, and X or each $R_5$ being from 8 to 40.

Substituents $R_1$, $R_2$, $R_3$ or X of these alpha oxyalkylene tertiary amines can be varied similar to the $R_1$, $R_2$, $R_3$ and X substituents, respectively, of the alpha tertiary amines. In addition to hydrogen, each $R_5$ hydrocarbyl group can be varied similar to the $R_1$ substituent and each $R_5$ alkylene oxide group can be varied similar to the $R_2$ and $R_3$ substituents.

Substituent $R_4$ can be any $C_2$—$C_6$ alkylene group. For ease of synthesis it is preferred that $R_4$ be a $C_2$—$C_3$ alkylene group, and even more preferably an ethylene group. The number of $C_2$—$C_6$ alkylene oxide units, n, is from 1 to 20, preferably from 1 to 10, and more preferably from 1 to 3. The most preferred tertiary amines herein are those in which n equals 1, since no additional stability is obtained for the respective amine oxide when n is greater than 1.

As with the alpha tertiary amines, it will be appreciated that the substituents for the alpha oxyalkylene tertiary amines should be selected such that the respective alpha oxyalkylene amine oxides exhibit sufficient surface activity and solubility for their intended use. Thus, the total number of carbon atoms for the hydrocarbyl and alkyl groups of $R_1$, $R_2$, $R_3$, and X or $R_5$ substituents should be from 8 to 40, preferably from 12 to 30. Additionally, when these tertiary amines, particularly the amide derivatives, have relatively long hydrocarbyl or alkyl chains at the various substituents, it is preferred that they also contain more than one $C_2$—$C_3$ alkylene (preferably ethylene) oxide unit for optimum solubility of the respective amine oxide,

especially in cold water. For example, n should be greater than 1 or one or more of the $R_2$, $R_3$, X or $R_4$ substituents should be a $C_2$—$C_3$ alkylene oxide group in such tertiary amines.

As used herein, the term "nonaromatic cyclic tertiary amine" refers to N-substituted monocyclic tertiary amines having the following general formula:

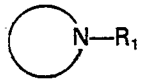

wherein $R_1$ is typically a $C_1$—$C_8$ alkyl group (e.g., alkyl, hydroxyalkyl), preferably a $C_1$—$C_4$ alkyl group and more preferably a methyl, ethyl, 2-hydroxyethyl or 2-hydroxypropyl group; and to bicyclic tertiary amines having the following general formula:

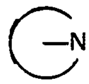

Such cyclic tertiary amines can include additional nitrogen atoms in the cyclic hydrocarbyl chain, as well as other hetero atoms such as oxygen. Examples of such cyclic tertiary amines which can be converted to the respective amine oxides by the process of the present application include quinuclidine, N-substituted 3-pyrrolines (e.g., N - methl - 3 - pyrroline), N-substituted piperidines (e.g., N-methylpiperidine), N-substituted morpholines (e.g., N-methylmorpholine), N-substituted tropines (e.g., N-methyltropine) and N,N'-substituted piperazines (e.g., N,N'-dimethylpiperazine).

B. Solvent systems containing tertiary amines

The tertiary amine to be oxidized is dissolved in an aqueous solvent system. It has been found that an aqueous environment is needed to stabilize the amine oxide formed during oxidation of the tertiary amine. It is believed that this stabilizing effect is due to the highly polar, protic nature of water as a solvent. For example, amine oxides tend to decompose in an aprotic solvents such as acetonitrile, thus significantly decreasing the yield of the amine oxide.

The concentration of the tertiary amine in the solvent system is usually dependent upon the molecular weight and solubility of the particular tertiary amine. For example, the concentration of a low-molecular weight, more soluble tertiary amine such as trimethyl amine usually ranges from 0.5 to 2.6 *M*. By contrast, the concentration of a higher molecular weight, less soluble tertiary amines such as dimethyldodecylamine usually ranges from 0.2 to 0.3 *M*. The concentration range for the tertiary amines is typically from 0.2 to 3.0 *M*. However, higher or lower concentrations can be employed consistent with the solubility of the particular tertiary amine.

For water-soluble tertiary amines such as trimethylamine, water can comprise the sole solvent of the solvent system. For other, substantially water-insoluble tertiary amines such as dimethyldodecylamine, a water-miscible solvent for the tertiary amine is used as well in the solvent system. Examples of suitable water-miscible solvents include the primary and secondary alcohols such as n-propyl alcohol, isopropyl alcohol, ethyl alcohol, and methyl alcohol. The ratio of the water-miscible solvent to water is dependent upon the particular tertiary amine and the concentration thereof in the aqueous solvent system. A weight ratio of water-miscible solvent:water of from 1:1 to 4:1 is usually sufficient, especially for the trialkyl tertiary amines such as triethylamine and dimethyldodecylamine.

Of particular importance to increasing the yield of amine oxide from the oxidation of the tertiary amine is the initial pH of the aqueous solvent system. While the pH of the solvent system throughout the reaction can be important to improving the yield of the amine oxide, the initial pH is particularly important. If the initial pH of the aqueous solvent system is too low, the nitrogen atom of the tertiary amine tends to be protonated. Such protonation inhibits conversion of the amine to the respective amine oxide.

To provide an effective yield of the amine oxide from the respective tertiary amine, the aqueous solvent system needs to have an initial pH about equal or greater than the pKa of the particular amine. As used herein, the term "about equal to or greater than" includes a pH below the pKa of the particular amine which does not significantly inhibit the oxidation of the amine to the amine oxide. Some pKa's of trialkyl tertiary amines are as follows:

| Amine | pKa |
|---|---|
| trimethyl | 9.8 |
| triethyl | 11.0 |

The initial pH should also not be so high as to promote side reactions, in particular alpha oxidation wherein the tertiary amine is converted to the respective secondary amine. Usually, an aqueous solvent system having an initial pH of from 9.5 to 12.5 is sufficient to provide conversion of trialkyl tertiary amines to the respective amine oxides in high yield. To maintain the pH of the solvent system in the desired range, a suitable buffer system can be used. Examples of suitable buffers include phosphate buffers, borate buffers and carbonate buffers. These buffers are also desirable from the standpoint of increasing the concentration of dissolved oxygen in the solvent system.

C. Reaction conditions for oxidation of tertiary amine to amine oxide with molecular oxygen

In the process of the present application, conversion of the tertiary amine to the respective amine oxide can be achieved through the use of molecular oxygen. Several sources of molecular oxygen can be employed. For example, air can be used in the process of the present application. Also, substantially pure oxygen can be used. Increasing the purity of the source of molecular oxygen will likewise increase the rate of conversion of the tertiary amine to the respective amine oxide.

The aqueous solvent system containing the tertiary amine is contacted with molecular oxygen in the absence of an oxidation catalyst, e.g., a metal complex catalyst. It has been found that the use of such a catalyst is unnecessary for converting the tertiary amine to the respective amine oxide using molecular oxygen. Included within the term "oxidation catalyst" are oxidation promoters which interact with molecular oxygen to form intermediate compounds (usually peroxy acids) containing active oxygen. Examples of such oxidation promoters are acetaldehyde, propionaldehyde and ozone.

An important parameter during this contacting step is the oxygen partial pressure. The oxygen partial pressure needs to be sufficient to insure that the concentration of molecular oxygen in the aqueous solvent system is sufficiently high to cause effective oxidation of the tertiary amine to the respective amine oxide. An oxygen partial pressure of at least 50 psi ($3.45\ 10^5$ Pa) has been found sufficient for this purpose. Usually, the partial pressure is at least 100 psi, ($6.9\ 10^5$ Pa) and is preferably at least 200 psi ($13.8\ 10^5$ Pa) with a range of from 200 to 1200 psi ($13.8\ 10^5$ to $82.810^5$ Pa) being typical. Generally, the higher the oxygen partial pressure, the greater will be the yield of amine oxide in a shorter reaction time.

Another important parameter during this contacting step is the temperature. The temperature needs to be high enough to provide sufficient activation energy to initiate the oxidation of the tertiary amine to the respective amine oxide. For this purpose, a temperature of at least 80°C has been found to be sufficient to provide effective oxidation of the tertiary amine by molecular oxygen to the respective amine oxide. In batch reactions, temperatures above 150°C can cause the formation of other reaction products over time due to decomposition of the amine oxide, particularly if it is relatively unstable. Preferably, the temperature for batch reactions is in the range of from 100° to 120°C. For continuous reaction systems, e.g., plug flow, temperatures above 150°C can be used due to the short time during which the amine oxide is subjected to such high temperatures.

For batch reactions, the yield of the amine oxide is usually dependent upon the duration of the reaction. As reaction time increases, the yield of amine oxide likewise increases. For example, after a reaction time of at least 16 hours, the yield of amine oxide from trialkyl tertiary amines is at least 20%, and is more typically at least 30%. After a reaction time of at least 64 hours, the yield of the amine oxide from such trialkyl tertiary amines is at least 40%, and is more typically at least 80%.

The oxidation of tertiary amines to the respective amine oxides using molecular oxygen according to the process of the present application can be carried out in a number of different batch reaction systems. An example of one such system is an autoclave (pressure bomb) which can be rocked to provide agitation. The aqueous solvent system containing the tertiary amine and any additional water-miscible solvent is poured into the batch reactor. The reactor is then opened to admit gaseous molecular oxygen from a source thereof, usually a compressed gas cylinder. The reactor is then pressurized to the appropriate oxygen partial pressure and heated to the appropriate temperature. The reactor is then allowed to run with agitation to insure adequate contact between the oxygen and solvent system until the tertiary amine is converted to the amine oxide in an appropriate yield.

The process of the present application can also be carried out in a continuous reaction system, e.g., plug flow type. In such a system, gaseous molecular oxygen as one reactant and the solvent system containing the tertiary amine as the other reactant are continuously admitted to a reaction chamber, usually in the form of a pipe. In this reaction chamber, the oxygen contacts the solvent system at the appropriate oxygen partial pressure and the appropriate temperature. The reaction mixture is then cooled at some point in the reaction chamber to quench the reaction. Such a system permits higher temperatures to be used which results in faster oxidation of the tertiary amine to the respective amine oxide. Also, the amine oxides, especially those which are relatively unstable, can be recovered before decomposition due to heat.

After the tertiary amine has been converted to the respective amine oxide in the appropriate yield, the amine oxide can be recovered from the aqueous solvent system by standard techniques. For example, the aqueous solvent system can be stripped away to recover the amine oxide. Activated carbon can also be added to the reaction mixture to remove other trace reaction products. If desired, the amine oxide can be recrystallized to increase the purity thereof.

Specific illustrations of oxidation of tertiary amines to amine oxides with molecular oxygen

The following examples of the oxidation of tertiary amines to amine oxides with molecular oxygen are used to illustrate the process of the present application:

All oxidations were carried out in a 0.5 liter stainless steel autoclave having a glass-liner. The solvent system containing distilled water, the particular tertiary amine and any additional water-miscible solvent were poured into the autoclave. The autoclave was then charged with air or pure oxygen from a compressed gas cylinder. The autoclave was then pressurized and heated to the appropriate oxygen partial pressure and temperature, respectively. The autoclave was rocked to insure adequate contact between the

oxygen and the solvent system. The reaction was allowed to run for the desired time. The amine oxide was recovered by stripping away the solvent system. The yield of amine oxide was determined by reverse phase high pressure liquid chromatography.

A. Tertiary amine

The oxidation of different tertiary amines by air or oxygen to the respective amine oxides is shown in the following Table:

TABLE I

| Amine (conc.) | $O_2$ Pressure (psi) | ($Pa \times 10^{-5}$) | Temp. (°C) | Reaction time (hrs) | Amine oxide yield (%) |
|---|---|---|---|---|---|
| $NMe_3$ (0.5 *M*) | 200 | 13.8 | 100 | 16 | 31.5 |
| $NEt_3$ (1.4 *M*) | 200 | 13.8 | 100 | 64 | 55 |
| $C_{12}NMe_2$ (0.025 *M*) | 200 | 13.8 | 100 | 16 | 46 |
| 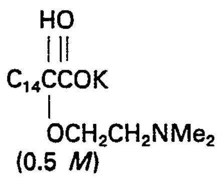 (0.5 *M*) | 100* | 6.9* | 100 | 16 | 10 |
| 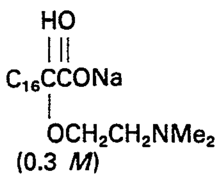 (0.3 *M*) | 200 | 13.8 | 100 | 16 | 40 |
| 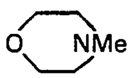 (0.2 *M*) | 1000* | 69* | 115 | 30 | 48 |
| Pyridine | 1000* | 69* | 100 | 16 | 0 |

*Pure oxygen

As can be seen in Table I, the process of the present application can be used to convert a variety of nonaromatic tertiary amines to the respective amine oxides. By comparison, aromatic tertiary amines such as pyridine are not converted by the process of the present application to the respective amine oxide.

B. Solvent system

The effect of different solvent systems on the oxidation of tertiary amines by air to the respective amine oxides at a pressure of 1000 psi (69 $10^5$ Pa) (oxygen partial pressure of 200 psi (13.8 $10^5$ Pa)) and a temperature of 100°C is shown in the following Table:

TABLE II

| Amine (conc.) | Solvent system | Ratio | Reaction time (hrs) | Amine oxide yield (%) |
|---|---|---|---|---|
| $NMe_3$ (2.4 *M*) | $H_2O$ | — | 16 | 33 |
| $NMe_3$* (1.0 *M*) | $CH_3CN$ | — | 16 | 11 |
| $NEt_3$** (1.4 *M*) | $H_2O$ | — | 64 | 43 |
| $NEt_3$ (1.4 *M*) | $MeOH:H_2O$ | 1:1 | 64 | 55 |
| $C_{12}NMe_2$** | $H_2O$ | — | 16 | Trace |
| $C_{12}NMe_2$ | $HCl:H_2O$ | pH 7 | 16 | 0 |
| $C_{12}NMe_2$ (0.25 *M*) | $MeOH:H_2O$ | 4:1 | 16 | 46 |
| $C_{12}NMe_2$ (0.24 *M*) | $MeOH:H_2O$ | 2:1 | 64 | 58 |
| $C_{12}NMe_2$ (0.27 *M*) | $EtOH:H_2O$ | 2:1 | 64 | 80 |

*Temp. 105°C, pure $O_2$ (1000 psi, 69 $10^5$ Pa)
**Two phases

As can be seen in Table II (see trimethylamine oxidations), use of an aqueous solvent system increases the yield of amine oxide. Also, for less water soluble tertiary amines (see triethylamine oxidations), inclusion of a water-miscible solvent increases the yield of amine oxide. Further, for substantially water insoluble tertiary amines (see dimethyldodecylamine oxidations), inclusion of a water-miscible solvent is necessary for any significant yield of amine oxide.

C. pH and Buffers

The effect of pH and buffers on the oxidation of trimethylamine (0.5 *M*) by air to trimethylamine oxide at a pressure of 1000 psi (69.$10^5$ Pa) (oxygen partial pressure of 200 psi (13.8 $10^5$ Pa)) and a temperature of 100°C for 16 hours is shown in the following Table:

TABLE III

| Buffer* | Buffer conc. | pH (Initial) | pH (Final) | Amine oxide yield (%) |
|---|---|---|---|---|
| None | — | 11.8 | 7.6 | 38 |
| None** | — | 2.2 | 3.1 | 0.4 |
| $PO_4$ | 1.4 *M* | 7.8 | 7.3 | 1.4 |
| $PO_4$ | 0.05 *M* | 12.0 | 9.3 | 29.4 |
| $PO_4$ | 0.02 *M* | 11.0 | 9.1 | 35.4 |
| $PO_4$ | 0.5 *M* | 12.3 | 9.8 | 49 |
| $BO_3$ | 1.1 *M* | 9.7 | 8.4 | 38.4 |
| $BO_3$ | 2.0 *M* | 10.1 | 8.9 | 32.6 |
| $CO_3$ | 1.1 *M* | 10.6 | 9.4 | 44.8 |

*$PO_4$=phosphate buffer; $BO_3$=borate buffer; $CO_3$=carbonate buffer
**1.0 *M* trimethylamine, pure oxygen (1200 psi, 82.8.$10^5$ Pa)

As can be seen in Table III, an initial pH about equal to or greater than the pKa of the tertiary amine (pKa of trimethylamine=9.8) results in a significant yield of amine oxide, while an initial pH significantly lower than the pKa results in a minimal yield of amine oxide. As can also be seen, various buffer systems can be used to maintain the pH of the solvent system during the reaction within a range providing a significant yield of amine oxide.

D. Oxygen partial pressure

The effect of different oxygen partial pressures on the oxidation of trimethylamine (0.5 *M*) to trimethylamine oxide at a temperature of 100°C for 16 hours is shown in the following Table:

TABLE IV

| Pressure (psi) | ($Pa \times 10^{-5}$) | Amine oxide yield (%) |
|---|---|---|
| 200 | 13.8 | 31.5 |
| 300 | 20.7 | 31.5 |
| 400 | 27.6 | 38 |
| 500* | 34.5* | 34.2 |

*Temp. 90°C

As can be seen in Table IV, oxygen partial pressures of 200 psi (13.8 $10^5$ Pa) and above provide effective oxidation of the tertiary amine to the respective amine oxide.

E. Temperature

The effect of temperature on the oxidation of trimethylamine (0.5 *M*) by air to trimethylamine oxide at a pressure of 1000 psi (69 $10^5$ Pa) (oxygen partial pressure of 200 psi (13.8 $10^5$ Pa)) for 16 hours is shown in the following Table:

TABLE V

| Temp. (°C) | Amine oxide yield (%) |
|---|---|
| 90 | 21.8 |
| 100 | 31.5 |
| 115 | 41 |
| 140 | 42.5 |

As can be seen in Table V, the amine oxide yield generally increases with increasing temperature. At 140°C, the yield reached a maximum with a darkening of the reaction mixture which indicates the formation of other reaction products due to decomposition of the amine oxide.

F. Duration of reaction

The effect of duration of the reaction on the oxidation of various tertiary amines by air to the respective amine oxides at a pressure of 1000 psi (69 $10^5$ Pa) (oxygen partial pressure of 200 psi (13.8 $10^5$ Pa)) and at a temperature of 100°C is shown in the following Table:

TABLE VI

| Amine (conc.) | Reaction time (hrs.) | Amine oxide yield (%) |
|---|---|---|
| $NMe_3$ (2.4 *M*) | 16 | 26.5 |
| $NMe_3$ (2.4 *M*) | 64 | 84 |
| $C_{12}NMe_2$ (0.25 *M*) | 16 | 46 |
| $C_{12}NMe_2$ (0.27 *M*) | 40 | 50 |
| $C_{12}NMe_2$ (0.21 *M*) | 64 | 85 |
| 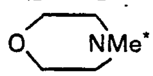 (0.2 *M*) | 7 | 22 |
| " | 30 | 48 |
| " | 72 | 62 |

*Temp. 115°C, pure $O_2$ (1000 psi, 69 $10^5$ Pa)

As can be seen in Table VI, increasing the duration of the reaction generally increases the yield of amine oxide for batch reactions.

**Claims**

1. A process for oxidizing a nonaromatic tertiary amine to the respective amine oxide, which comprises the steps of:

(a) providing an aqueous solvent system containing a nonaromatic tertiary amine, the aqueous solvent system having an initial pH about equal to or greater than the pKa of the tertiary amine; and

(b) contacting the aqueous solvent system with molecular oxygen in the absence of an oxidation catalyst at an oxygen partial pressure of at least 50 psi (3.45 $10^5$ Pa) and at a temperature of at least 80°C to oxidize the tertiary amine to the respective amine oxide.

2. A process according to Claim 1 wherein the tertiary amine is a noncyclic tertiary amine.

3. A process according to Claim 2 wherein the noncyclic tertiary amine has the formula:

$$R_1(OCH_2H_4)_n{-}\underset{\displaystyle R_3}{\overset{\displaystyle R_2}{N}}$$

wherein $R_1$ is a $C_{10}$—$C_{22}$ hydrocarbyl group and n is from 0 to 10; and $R_2$ and $R_3$ are each a $C_1$—$C_4$ alkyl group.

4. A process according to Claim 3 wherein $R_1$ is a $C_{10}$—$C_{18}$ alkyl group and n is 0.

5. A process according to Claim 4 wherein $R_1$ is a dodecyl group and wherein $R_2$ and $R_3$ are both methyl groups.

6. A process according to Claim 2 wherein the noncyclic tertiary amine has the formula

$$R_1{-}\underset{\displaystyle N{-}R_2 \atop \displaystyle R_3}{CH}\overset{O}{\overset{\|}{C}}OX$$

wherein $R_1$ is hydrogen, or a $C_1$—$C_{20}$ hydrocarbyl group; $R_2$ and $R_3$ are each a $C_1$—$C_{20}$ alkyl group, or a $C_2$—$C_3$ alkylene oxide group having from 1 to 10 alkylene oxide units; and X is hydrogen or a water-soluble metal, ammonium or substituted ammonium cation; the total number of carbon atoms for the hydrocarbyl and alkyl groups of $R_1$, $R_2$ and $R_3$ being from 8 to 36.

7. A process according to Claim 6 wherein $R_1$ is a $C_{10}$—$C_{16}$ alkyl group; $R_2$ and $R_3$ are each a $C_1$—$C_4$ alkyl group, or an ethylene oxide group having from 1 to 5 ethylene oxide units; and X is a water-soluble metal cation.

8. A process according to Claim 2 wherein the noncyclic tertiary amine has the formula:

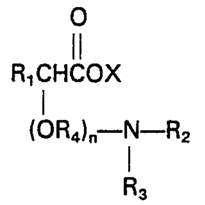

or

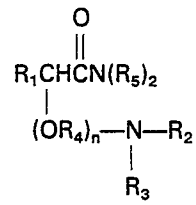

wherein $R_1$ is hydrogen, or a $C_1$—$C_{20}$ hydrocarbyl group; $R_2$ and $R_3$ are each a $C_1$—$C_{20}$ alkyl group, or a $C_2$—$C_3$ alkylene oxide group having from 1 to 10 alkylene oxide units; $R_4$ is a $C_2$—$C_6$ alkylene group, and n is from 1 to 20; each $R_5$ is hydrogen, a $C_1$—$C_{20}$ hydrocarbyl group, or a $C_2$—$C_3$ alkylene oxide group having from 1 to 10 alkylene oxide units; and X is hydrogen, a water-soluble metal, ammonium or substituted ammonium cation, a $C_1$—$C_8$ hydrocarbyl group, or a $C_2$—$C_3$ alkylene oxide group having from 1 to 10 alkylene oxide units; the total number of carbon atoms for the hydrocarbyl and alkyl groups of $R_1$, $R_2$, $R_3$ and X or each $R_5$ being from 8 to 40.

9. A process according to Claim 8 wherein $R_1$ is a $C_{10}$—$C_{16}$ alkyl group; $R_2$ and $R_3$ are each a $C_1$—$C_4$ alkyl group, or an ethylene oxide group having from 1 to 5 ethylene oxide units; $R_4$ is a $C_2$—$C_3$ alkylene group and n is from 1 to 10; and X is a water-soluble metal cation.

10. A process according to Claim 2 wherein the noncyclic tertiary amine is selected from the group consisting of trimethylamine and triethylamine.

11. A process according to Claim 1 wherein the tertiary amine is a nonaromatic cyclic tertiary amine.

12. A process according to Claim 11 wherein the cyclic tertiary amine is selected from the group consisting of quinuclidine, N-substituted 3-pyrrolines, N-substituted piperidines, N-substituted morpholines, N-substituted tropines and N,N'-substituted piperazines.

13. A process according to Claim 1 wherein the partial pressure during said contacting step is at least 100 psi (6.9 $10^5$ Pa).

14. A process according to Claim 13 wherein the partial pressure during said contacting step is at least 200 psi (13.8 $10^5$ Pa).

15. A process according to Claim 13 wherein the temperature during said contacting step is from 80° to 150°C.

16. A process according to Claim 15 wherein the temperature during said contacting step is from 100° to 120°C.

17. A process according to Claim 13 wherein the concentration of the tertiary amine in the aqueous solvent system prior to said contacting step is from 0.2 to 3.0 *M*.

18. A process according to Claim 13 wherein the initial pH is from 9.5 to 12.5.

19. A process according to Claim 13 wherein the aqueous solvent system is contacted with air.

20. A process according to Claim 13 wherein the aqueous solvent system is contacted with substantially pure oxygen.

21. A process according to Claim 13 wherein the aqueous solvent system further comprises a water-miscible solvent for the tertiary amine.

22. A process according to Claim 21 wherein the water-miscible solvent is selected from the group consisting of methyl alcohol and ethyl alcohol.

23. A process according to Claim 22 wherein the weight ratio of water-miscible solvent to water is from 1:1 to 4:1.

24. A process according to Claim 1 comprising the further step of recovering the amine oxide from the aqueous solvent system.

**Patentansprüche**

1. Verfahren zur Oxidation eines nichtaromatischen tertiären Amins zu dem entsprechenden Aminoxid, dadurch gekennzeichnet, daß es die Stufen:

(a) Bereitstellung eines wäßrigen Lösungsmittelsystems mit einem Gehalt eines nichtaromatischen tertiären Amins, wobei das wäßrige Lösungsmittelsystem einen anfänglichen pH-Wert aufweist, der etwa gleich wie oder größer als der pKa-Wert des tertiären Amins ist; und

(b) In-Berührungbringen des wäßrigen Lösungsmittelsystems mit molekularem Sauerstoff in Abwesenheit eines Oxidationskatalysators bei einem Sauerstoffpartialdruck von mindestens $3{,}45 \times 10^5$ Pa (50 psi) und einer Temperatur von mindestens 80°C zur Oxidation des tertiären Amins zu dem entsprechenden Aminoxid umfaßt.

2. Verfahren gemäß Anspruch 1, worin das tertiäre Amin ein nicht zyklisches tertiäres Amin ist.

3. Verfahren gemäß Anspruch 2, worin das nicht zyklische tertiäre Amin die Formel:

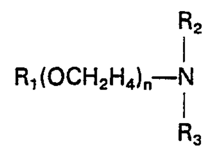

aufweist, worin $R_1$ eine $C_{10}$—$C_{22}$-Kohlenwasserstoffgruppe darstellt und n 0 bis 10 bedeutet; und $R_2$ und $R_3$ jeweils eine $C_1$—$C_4$-Alkylgruppe bedeuten.

4. Verfahren gemäß Anspruch 3, worin $R_1$ eine $C_{10}$—$C_{18}$-Alkylgruppe darstellt und n 0 bedeutet.

5. Verfahren gemäß Anspruch 4, worin $R_1$ eine Dodecylgruppe ist und worin $R_2$ und $R_3$ beide Methylgruppen darstellen.

6. Verfahren gemäß Anspruch 2, worin das nicht zyklische tertiäre Amin die Formel

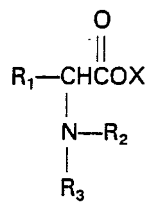

aufweist, worin $R_1$ Wasserstoff oder eine $C_1$—$C_{20}$-Kohlenwasserstoffgruppe bedeutet; $R_2$ und $R_3$ jeweils eine $C_1$—$C_{20}$-Alkylgruppe oder eine $C_2$—$C_3$-Alkylenoxidgruppe mit 1 bis 10 Alkylenoxideinheiten darstellen; und X Wasserstoff oder ein wasserlösliches Metall-, Ammonium- oder substituiertes Ammoniumkation bedeutet; wobei die Gesamtzahl der Kohlenstoffatome für die Kohlenwasserstoff- und Alkylgruppen von $R_1$, $R_2$ und $R_3$ 8 bis 36 beträgt.

7. Verfahren gemäß Anspruch 6, worin $R_1$ eine $C_{10}$—$C_{16}$-Alkylgruppe ist, $R_2$ und $R_3$ jeweils eine $C_1$—$C_4$-Alkylgruppe oder eine Ethylenoxidgruppe mit 1 bis 5 Äthylenoxideinheiten sind; und X ein wasserlösliches Metallkation bedeutet.

8. Verfahren gemäß Anspruch 2, worin das nicht zyklische tertiäre Amin die Formel

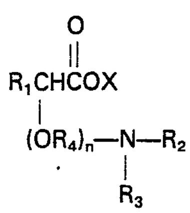

oder

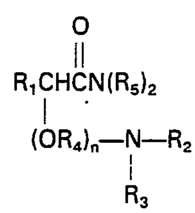

aufweist, worin $R_1$ Wasserstoff oder eine $C_1$—$C_{20}$-Kohlenwasserstoffgruppe bedeutet; $R_2$ und $R_3$ jeweils eine $C_1$—$C_{20}$-Alkylgruppe oder eine $C_2$—$C_3$-Alkylenoxidgruppe mit 1 bis 10 Alkylenoxideinheiten bedeuten; $R_4$ eine $C_2$—$C_6$-Alkylengruppe ist und n 1 bis 20 bedeutet; jeder Rest $R_5$ Wasserstoff, eine $C_1$—$C_{20}$-Kohlenwasserstoffgruppe oder eine $C_2$—$C_3$-Alkylenoxidgruppe mit 1 bis 10 Alkylenoxideinheiten ist; und X Wasserstoff, ein wasserlösliches Metall-, Ammonium- oder substituiertes Ammoniumkation, eine $C_1$—$C_8$-Kohlenwasserstoffgruppe oder eine $C_2$—$C_3$-Alkylenoxidgruppe mit 1 bis 10 Alkylenoxideinheiten darstellt; wobei die Gesamtzahl der Kohlenstoffatome für die Kohlenwasserstoff- und Alkylgruppen von $R_1$, $R_2$, $R_3$ und X oder jeden Rest $R_5$ 8 bis 40 ist.

9. Verfahren gemäß Anspruch 8, worin $R_1$ eine $C_{10}$—$C_{16}$-Alkylgruppe ist; $R_2$ und $R_3$ jeweils eine $C_1$—$C_4$-Alkylgruppe oder eine Ethylenoxidgruppe mit 1 bis 5 Ethylenoxideinheiten darstellt; $R_4$ eine $C_2$—$C_3$-Alkylengruppe ist und n 1 bis 10 bedeutet; und X ein wasserlösliches Metallkation darstellt.

10. Verfahren gemäß Anspruch 2, worin das nicht zyklische tertiäre Amin ausgewählt ist aus der Gruppe bestehende aus Trimethylamin und Triethylamin.

11. Verfahren gemäß Anspruch 1, worin das tertiäre Amin ein nichtaromatisches zyklisches tertiäres Amin ist.

12. Verfahren gemäß Anspruch 11, worin das zyklische tertiäre Amin ausgewählt ist aus der Gruppe bestehend aus Chinuclidinen, N-substituierten 3-Pyrrolinen, N-substituierten Piperidinen, N-substituierten Morpholinen, N-substituierten Tropinen und N,N'-substituierten Piperazinen.

13. Verfahren gemäß Anspruch 1, worin der Partialdruck während der Berührungsstufe mindestens $6{,}9\times10^5$ Pa (100 psi) ist.

14. Verfahren gemäß Anspruch 13, worin der Partialdruck während der Berührungsstufe mindestens $13{,}8\times10^5$ Pa (200 psi) ist.

15. Verfahren gemäß Anspruch 13, worin die Temperatur während der Berührungsstufe 80 bis 150°C beträgt.

16. Verfahren gemäß Anspruch 13, worin die Temperatur während der Berührungsstufe 100 bis 120°C beträgt.

17. Verfahren gemäß Anspruch 13, worin die Konzentration des tertiären Amins in dem wäßrigen Lösungsmittelsystem vor der Berührungsstufe 0,2 bis 3,0 M beträgt.

18. Verfahren gemäß Anspruch 13, worin der anfängliche pH-Wert 9,5 bis 12,5 beträgt.

19. Verfahren gemäß Anspruch 13, worin das wäßrige Lösungsmittelsystem mit Luft in Berührung gebracht wird.

20. Verfahren gemäß Anspruch 13, worin das wäßrige Lösungsmittelsystem mit im wesentlichen reinem Sauerstoff in Berührung gebracht wird.

21. Verfahren gemäß Anspruch 13, worin das wäßrige Lösungsmittelsystem weiterhin ein mit Wasser mischbares Lösungsmittel für das tertiäre Amin umfaßt.

22. Verfahren gemäß Anspruch 21, worin das mit Wasser mischbare Lösungsmittel ausgewählt ist aus der Gruppe bestehend aus Methylalkohol und Ethylalkohol.

23. Verfahren gemäß Anspruch 22, worin das Gewichtsverhältnis des mit Wasser mischbaren Lösungsmittels zu Wasser 1:1 bis 4:1 beträgt.

24. Verfahren gemäß Anspruch 1, umfassend die weitere Stufe der Gewinnung des Aminoxides aus dem wäßrigen Lösungsmittelsystem.

**Revendications**

1. Procédé pour l'oxydation d'une amine tertiaire non-aromatique en l'oxyde d'amine correspondant, qui comprend les étapes consistant

(a) à former un système solvant aqueux contenant une amine tertiaire non-aromatique, le système solvant aqueux ayant un pH initial environ égal ou supérieur au pKa de l'amine tertiaire; et

(b) à mettre en contact le système solvant aqueux avec de l'oxygène moléculaire, en l'absence de catalyseur d'oxydation, à une pression partielle d'oxygène d'au moins 3,45 $10^6$ Pa (50 psi), et à une température d'au moins 80°C, pour oxyder l'amine tertiaire en l'oxyde d'amine correspondant.

2. Procédé selon la revendication 1, dans lequel l'amine tertiaire est une amine tertiaire acyclique.

3. Procédé selon la revendication 2, dans lequel l'amine tertiaire acyclique a la formule

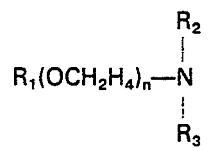

dans laquelle $R_1$ est un groupe hydrocarboné en $C_{10}$—$C_{22}$ et n vaut de 0 à 10; et $R_2$ et $R_3$ sont chacun un groupe alkyle en $C_1$—$C_4$.

4. Procédé selon la revendication 3, dans lequel $R_1$ est un groupe alkyle en $C_{10}$—$C_{18}$ et n vaut 0.

5. Procédé selon la revendication 4, dans lequel $R_1$ est un groupe dodécyle et dans lequel $R_2$ et $R_3$ sont tous les deux des groupes méthyle.

6. Procédé selon la revendication 2, dans lequel l'amine tertiaire acyclique a la formule:

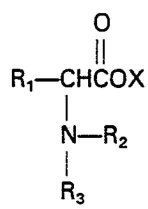

dans laquelle $R_1$ est l'hydrogène ou un groupe hydrocarboné en $C_1$—$C_{20}$; $R_2$ et $R_3$ sont chacun un groupe alkyle en $C_1$—$C_{20}$ ou un groupe oxyde d'alkylène en $C_2$—$C_3$ ayant de 1 à 10 motifs oxyde d'alkylène; et X est l'hydrogène ou un cation métallique, ammonium ou ammonium substitué soluble dans l'eau; le nombre total d'atomes de carbone pour les groupes hydrocarboné et alkyle de $R_1$, $R_2$ et $R_3$ étant de 8 à 36.

7. Procédé selon la revendication 6, dans lequel $R_1$ est un groupe alkyle en $C_{10}$—$C_{16}$; $R_2$ et $R_3$ sont chacun un groupe alkyle en $C_1$—$C_4$, ou un groupe oxyde d'éthylène ayant de 1 à 5 motifs oxyde d'éthylène; et X est un cation métallique soluble dans l'eau.

8. Procédé selon la revendication 2, dans lequel l'amine tertiaire acyclique a la formule:

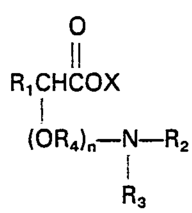

ou

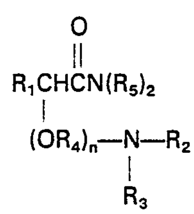

dans lesquelles $R_1$ est l'hydrogène, ou un groupe hydrocarboné en $C_1$—$C_{20}$; $R_2$ et $R_3$ sont chacun un groupe alkyle en $C_1$—$C_{20}$, ou un groupe oxyde d'alkylène en $C_2$—$C_3$ ayant de 1 à 10 motifs oxyde d'alkylène; $R_4$ est un groupe alkylène en $C_2$—$C_6$, et n est 1 à 20; chaque radical $R_5$ est l'hydrogène, un groupe hydrocarboné en $C_1$—$C_{20}$ ou un groupe oxyde d'alkylène en $C_2$—$C_3$ ayant de 1 à 10 motifs oxyde d'alkylène; et X est l'hydrogène, un cation métallique, ammonium ou ammonium substitué soluble dans l'eau, un groupe hydrocarboné en $C_1$—$C_8$ ou un groupe oxyde d'alkylène en $C_2$—$C_3$ ayant de 1 à 10 motifs oxyde d'alkylène; le nombre total d'atomes de carbone pour les groupes hydrocarboné et alkyle de $R_1$, $R_2$, $R_3$ et X, ou de chaque radical $R_5$, étant de 8 à 40.

9. Procédé selon la revendication 8, dans lequel $R_1$ est un groupe alkyle en $C_{10}$—$C_{16}$; $R_2$ et $R_3$ sont chacun un groupe alkyle en $C_1$—$C_4$ ou un groupe oxyde d'éthylène ayant de 1 à 5 motifs oxyde d'éthylène; $R_4$ est un groupe alkylène en $C_2$—$C_3$ et n est 1 à 10; et X est un cation métallique soluble dans l'eau.

10. Procédé selon la revendication 2, dans lequel l'amine tertiaire acyclique est choisie dans le groupe comprenant la triméthylamine et la triéthylamine.

11. Procédé selon la revendication 1, dans lequel l'amine tertiaire est une amine tertiaire cyclique non-aromatique.

12. Procédé selon la revendication 11, dans lequel l'amine tertiaire cyclique est choisie dans le groupe comprenant la quinuclidine, les 3-pyrrolidines N-substituées, les pipéridines N-substituées, les morpholines N-substituées, les tropines N-substituées et les pipérazines N,N'-substituées.

13. Procédé selon la revendication 1, dans lequel la pression partielle, pendant ladite étape de mise en contact, est d'au moins 6,9 $10^5$ Pa (100 psi).

14. Procédé selon la revendication 13, dans lequel la pression partielle au cours de ladite étape de mise en contact est d'au moins 13,8 $10^5$ Pa (200 psi).

15. Procédé selon la revendication 13, dans lequel la température au cours de ladite étape de mise en contact est de 80 à 150°C.

16. Procédé selon la revendication 15, dans lequel la température au cours de ladite étape de mise en contact est de 100 à 120°C.

17. Procédé selon la revendication 13, dans lequel la concentration de l'amine tertiaire dans le système solvant aqueux avant ladite étape de mise en contact est de 0,2 à 0,3 M.

18. Procédé selon la revendication 13, dans lequel le pH initial est de 9,5 à 12,5.

19. Procédé selon la revendication 13, dans lequel le système solvant aqueux est mis en contact avec l'air.

20. Procédé selon la revendication 13, dans lequel le système solvant aqueux est mis en contact avec de l'oxygène pratiquement pur.

21. Procédé selon la revendication 13, dans lequel le système solvant aqueux comprend en outre un solvant de l'amine tertiaire miscible à l'eau.

22. Procédé selon la revendication 21, dans lequel le solvant miscible à l'eau est choisi dans le groupe comprenant l'alcool méthylique et l'alcool éthylique.

23. Procédé selon la revendication 22, dans lequel le rapport pondéral entre le solvant miscible à l'eau et l'eau est de 1:1 à 4:1.

24. Procédé selon la revendication 1, comprenant l'étape supplémentaire consistant à séparer l'oxyde d'amine du système solvant aqueux.